Europäisches Patentamt

European Patent Office

Office européen des brevets

(11) Publication number: **0 428 266 B1**

# EUROPEAN PATENT SPECIFICATION

(45) Date of publication of patent specification: **28.09.94** (51) Int. Cl.⁵: **C07K 13/00**, A61K 37/02

(21) Application number: **90311189.6**

(22) Date of filing: **12.10.90**

(54) Anticancer agent.

(30) Priority: **13.10.89 JP 265049/89**
**26.06.90 JP 165727/90**
**17.07.90 JP 187137/90**

(43) Date of publication of application:
**22.05.91 Bulletin 91/21**

(45) Publication of the grant of the patent:
**28.09.94 Bulletin 94/39**

(84) Designated Contracting States:
**DE FR GB IT**

(56) References cited:
EP-A- 0 207 751      EP-A- 0 329 413
EP-A- 0 349 342      EP-A- 0 376 491
EP-A- 0 399 806      WO-A-90/13306

CHEMICAL ABSTRACTS, vol. 113, 1990, page
561, abstract no. 57494v, Columbus,Ohio, US

(73) Proprietor: **TAKARA SHUZO CO. LTD.**
**609 Takenakacho**
**Fushimi-ku Kyoto (JP)**

(72) Inventor: **Azuma, Ichiro**
**5-3-2, Makomanai Kamimachi,**
**Minami-ku**
**Sapporo-shi, Hokkaido (JP)**
Inventor: **Saiki, Ikuo**
**3-6-7-45, Hakken 3-jyo Nishi,**
**Nishi-ku**
**Sapporo-shi, Hokkaido (JP)**
Inventor: **Kimizuka, Fusao**
**1500-20, Furukawa-cho**
**Ohmihachiman-shi, Shiga-ken (JP)**
Inventor: **Kato, Ikunoshin**
**1-1-150, Nanryo-cho**
**Uji-shi, Kyoto-fu (JP)**

(74) Representative: **Geering, Keith Edwin et al**
**REDDIE & GROSE**
**16 Theobalds Road**
**London WC1X 8PL (GB)**

**Description**

This invention relates to the use of a particular polypeptide for the manufacture of a medicament for the therapeutic application of cancer. The polypeptide corresponds to a particular functional domain in the fibronectin (hereinafter referred to as FN) molecule, and it has physiological activities.

There are a variety of anticancer agents, including alkylating agents, anticancer antibiotics, antimetabolites and alkaloids. At present, there are several dozens of anticancer agents which can be used clinically.

However, in general, these anticancer agents are toxic and have other strong side effects, and the clinical effects cannot be further improved because of these drawbacks.

This invention utilizes substances present physiologically in the body to provide the use of a polypeptide of formula [IV] (below) for the manufacture of a medicament for the therapeutic application of cancer, with higher safety than has been obtained before.

A known anticancer agent contains a pharmacologically effective amount of compound of the following formula [I]:

Y-(X)m-Z     [I]

wherein Y is a hydrogen atom or a polypeptide corresponding to or functionally equivalent to the polypeptide of the cell-binding domain of the FN molecule, X is a spacer,
m is 0 or 1, and Z is -OH or a polypeptide corresponding to or functionally equivalent to the polypeptide of the heparin-binding domain of the FN molecule, at least one of Y and Z being said polypeptide.

The spacer is an insertion sequence which is used to adjust the distance between the polypeptide molecule or between Y and Z. Any amino acid, polypeptide, or derivative of the like can be used as the spacer provided that it has no effect on the activity of the polypeptide(s)

The presence of this spacer does not affect the effect to be obtained by the use of the polypeptide and it is possible to remove the spacer readily by use of the methods of site-specific mutagenesis.

During the research work by the inventors of this invention on the functional domains of FN, which is a physiological substance, it has been found that polypeptides with the structure of the Formula [I] have a strong anticancer activity.

FN is a dimer made of polypeptides with an S-S bond in the vicinity of the C-terminal and with a molecular weight of about 500,000. Its sequence of amino acids has repeating structures named types I, II and III. It also has domains with a variety of functions such as binding activity with cells, collagen, heparin, fibrin, and so on.

We have done research work on the novel use of the polypeptide of the functional domains of FN and as a result have found that the cell-binding polypeptide, the heparin-binding polypeptide, and the chimera polypeptide of the cell-binding polypeptide and the heparin-binding polypeptide all have strong anticancer activity.

Examples of the polypeptides which correspond to or are functionally equivalent to the polypeptide of the cell-binding domain of the FN molecule, which polypeptides have cell-binding activity include those polypeptides with cell-binding activity disclosed in our Japanese Laid-Open Patent Applications Nos. 180900/89 (U.S. Patent No. 5049658), 206998/89 (U.S. Patent No. 5045631), 97397/90 (U.S. Patent No. 5102988) and 152990/90 (U.S. Patent No. 5136023). These polypeptides with cell-binding activity can be prepared efficiently by the methods of genetic engineering.

Below, an example will be given in detail concerning the polypeptide with 274 amino acid residues.

The polypeptide $Pro^{1239}$-$Asp^{1512}$ with 274 amino acid residues has the structure shown in the following Formula [II]:

```
       1 2 3 9
     Pro Thr Asp Leu Arg Phe Thr Asn Ile Gly
     Pro Asp Thr Met Arg Val Thr Trp Ala Pro
     Pro Pro Ser Ile Asp Leu Thr Asn Phe Leu
     Val Arg Tyr Ser Pro Val Lys Asn Glu Glu
     Asp Val Ala Glu Leu Ser Ile Ser Pro Ser
     Asp Asn Ala Val Val Leu Thr Asn Leu Leu
     Pro Gly Thr Glu Tyr Val Val Ser Val Ser
     Ser Val Tyr Glu Gln His Glu Ser Thr Pro
     Leu Arg Gly Arg Gln Lys Thr Gly Leu Asp
     Ser Pro Thr Gly Ile Asp Phe Ser Asp Ile
     Thr Ala Asn Ser Phe Thr Val His Trp Ile
     Ala Pro Arg Ala Thr Ile Thr Gly Tyr Arg
     Ile Arg His His Pro Glu His Phe Ser Gly
     Arg Pro Arg Glu Asp Arg Val Pro His Ser
     Arg Asn Ser Ile Thr Leu Thr Asn Leu Thr
     Pro Gly Thr Glu Tyr Val Val Ser Ile Val
     Ala Leu Asn Gly Arg Glu Glu Ser Pro Leu
     Leu Ile Gly Gln Gln Ser Thr Val Ser Asp
     Val Pro Arg Asp Leu Glu Val Val Ala Ala
     Thr Pro Thr Ser Leu Leu Ile Ser Trp Asp


     Ala Pro Ala Val Thr Val Arg Tyr Tyr Arg
     Ile Thr Tyr Gly Glu Thr Gly Gly Asn Ser
     Pro Val Gln Glu Phe Thr Val Pro Gly Ser
     Lys Ser Thr Ala Thr Ile Ser Gly Leu Lys
     Pro Gly Val Asp Tyr Thr Ile Thr Val Tyr
     Ala Val Thr Gly Arg Gly Asp Ser Pro Ala
     Ser Ser Lys Pro Ile Ser Ile Asn Tyr Arg
                      1 5 1 2
     Thr Glu Ile Asp                     · · · [II]
```

Its method of preparation is described in Japanese Laid-Open Patent Application No. 97397/90 (U.S. Patent No. 5102988) and will be concretely explained below.

In this specification, the numbers used to label the amino acids are the numbers of the amino acid residues from the N-terminal of the amino acid sequence obtained by translation of the cDNA sequence of FN in the EMBL Data Bank.

EP 0 428 266 B1

The polypeptide $Pro^{1239}$-$Asp^{1512}$ with 274 amino acid residues is the same as the polypeptide $Pro^{1239}$-$Met^{1517}$ with 279 amino acid residues for which a patent has already been applied (Japanese Laid-Open Patent Application No. 206998/89: U.S. Patent No. 5045631), except that five amino acid residues on the C-terminal have been deleted. The preparation of the polypeptide $Pro^{1239}$-$Asp^{1512}$ with 274 amino acid residues can be done readily with the use of the plasmid pTFD707 which codes for the polypeptide $Pro^{1239}$-$Met^{1517}$ with 279 amino acid residues. A plasmid coding for the polypeptide $Pro^{1239}$-$Asp^{1512}$ with 274 amino acid residues can be prepared by the replacement of the codon AAA for $Lys^{1513}$, which is the fifth residue from the C-terminal of the polypeptide with 279 amino acid residues, with the stop codon TAA. For this purpose, the methods of site-specific mutagenesis can be used.

Cells of Escherichia coli JM109/pTF7221 into which a plasmid that expresses the polypeptide $Pro^{1239}$-$Asp^{1512}$ with 274 amino acid residues has been introduced have been deposited at the Fermentation Research Institute of the Agency of Industrial Science and Technology, Japan, as FERM BP-1915.

The purification of the polypeptide with cell-binding activity from the recombinant cells can be done, for example, as follows. The cell pellet is suspended in a buffer, the suspension is sonicated, and the soluble and insoluble fractions are separated. The insoluble fraction is solubilized in a buffer that contains 7 M urea. These two soluble fractions are pooled and put on a column of Sepharose 4B bound with antibody specific for the cell-binding domain of FN for purification by affinity chromatography. Elution is done with a buffer with a pH in the vicinity of 2.3. Immunoblotting is used to obtain the desired fraction, by which means the polypeptide with cell-binding activity is obtained. When necessary, FPLC and HPLC can be used for the purification.

An example of the polypeptide which corresponds to or is functionally equivalent to the polypeptide of the heparin-binding domain of the FN molecule, which polypeptide has heparin-binding activity includes the polypeptide with heparin-binding activity disclosed in our Japanese Patent Application No. 131453/89 (U.S. Patent No. 5198423). This application discloses the polypeptide $Pro^{1239}$-$Ser^{1515}$-$Met$-$Ala^{1690}$-$Thr^{1960}$, of formula [IV], below.

The polypeptide with heparin-binding activity can be prepared efficiently by the methods of genetic engineering. Below, an example will be given in detail concerning the polypeptide with 271 amino acid residues.

The polypeptide $Ala^{1690}$-$Thr^{1960}$ with 271 amino acid residues has the structure shown in the following Formula [III]:

```
1690
Ala Ile Pro Ala Pro Thr Asp Leu Lys Phe
Thr Gln Val Thr Pro Thr Ser Leu Ser Ala
Gln Trp Thr Pro Pro Asn Val Gln Leu Thr
Gly Tyr Arg Val Arg Val Thr Pro Lys Glu
Lys Thr Gly Pro Met Lys Glu Ile Asn Leu
Ala Pro Asp Ser Ser Ser Val Val Val Ser
Gly Leu Met Val Ala Thr Lys Tyr Glu Val
Ser Val Tyr Ala Leu Lys Asp Thr Leu Thr
Ser Arg Pro Ala Gln Gly Val Val Thr Thr
Leu Glu Asn Val Ser Pro Pro Arg Arg Ala
Arg Val Thr Asp Ala Thr Glu Thr Thr Ile
Thr Ile Ser Trp Arg Thr Lys Thr Glu Thr
Ile Thr Gly Phe Gln Val Asp Ala Val Pro
Ala Asn Gly Gln Thr Pro Ile Gln Arg Thr
Ile Lys Pro Asp Val Arg Ser Tyr Thr Ile
Thr Gly Leu Gln Pro Gly Thr Asp Tyr Lys
Ile Tyr Leu Tyr Thr Leu Asn Asp Asn Ala
Arg Ser Ser Pro Val Val Ile Asp Ala Ser
Thr Ala Ile Asp Ala Pro Ser Asn Leu Arg
Phe Leu Ala Thr Thr Pro Asn Ser Leu Leu
Val Ser Trp Gln Pro Pro Arg Ala Arg Ile
Thr Gly Tyr Ile Ile Lys Tyr Glu Lys Pro
Gly Ser Pro Pro Arg Glu Val Val Pro Arg
Pro Arg Pro Gly Val Thr Glu Ala Thr Ile
Thr Gly Leu Glu Pro Gly Thr Glu Tyr Thr
Ile Tyr Val Ile Ala Leu Lys Asn Asn Gln
Lys Ser Glu Pro Leu Ile Gly Arg Lys Lys
1960
Thr        · · ·  [III]-
```

Its method of preparation is disclosed in our Japanese Patent Application No. 131453/89 (U.S. Patent No. 5198423) , and will be explained in detail below.

The plasmid pLF2435 which carries the cDNA fragment coding for the heparin-binding domain of human FN is obtained by the reconstruction of the plasmids pLF2, pLF3, pLF4 and pLF5 described in Biochemistry, vol. 25, 4936-4941 (1986).

The desired cDNA fragment is cleaved from this plasmid pLF2435 by the use of restriction enzymes, and DNA ligase is used to link synthetic DNA that contains an initiation codon at its 5' end and synthetic DNA that contains a termination codon at its 3' end. Then, by linkage with an appropriate expression vector, it is possible to construct a plasmid pHD101 that expresses the polypeptide $Ala^{1690}$-$Thr^{1960}$ that has the

271 amino acid residues of the heparin-binding domain.

As the expression vector, any of the known expression vectors can be used. For example, it is possible to obtain satisfactory results with the use of pUC118N/pUC119N (FEBS Letters, vol. 223, 174-180, 1987) or its derivatives. By the introduction of this plasmid into cells of E. coli, the heparin-binding polypeptide is expressed, and its properties can be investigated.

Cells of E. coli HB101/pHD101 into which a plasmid that expresses the polypeptide Ala$^{1690}$-Thr$^{1960}$ with 271 amino acid residues has been introduced have been deposited at the Fermentation Research Institute of the Agency of Industrial Science and Technology, Japan, as FERM BP-2264.

The purification of the polypeptide with heparin-binding activity from the recombinant cells can be done, for example, as follows. Recombinant E. coli cells are cultured in a medium such as L-broth and the cells are collected and treated by sonication. The sonicated cell extract is centrifuged, and the supernatant is obtained. The supernatant is first dialyzed and then passed through a DEAE ion-exchange column. The eluent is then treated by CM ion-exchange chromatography and/or affinity chromatography on heparin-agarose, or the like.

A chimera polypeptide made of the polypeptide with cell-binding activity and the polypeptide with heparin-binding activity is a polypeptide which contains the polypeptide corresponding to or equivalent functionally to the polypeptide of the cell-binding domain of the FN molecule bound directly or by means of a spacer to the polypeptide corresponding to or equivalent functionally to the polypeptide of the heparin-binding domain of the FN molecule.

In making an example of such a polypeptide, the inventors of this invention have used the methods of genetic engineering to produce efficiently a polypeptide with 549 amino acid residues that is disclosed in the detailed description of our Japanese Patent Application No. 131453/89 (U.S. Patent No. 5198423).

The polypeptide with 549 amino acid residues will be described in detail below.

The polypeptide Pro$^{1239}$-Ser$^{1515}$-Met-Ala$^{1690}$-Thr$^{1960}$ with 549 amino acid residues is of the following Formula [IV]:

```
Pro¹²³⁹ Thr  Asp  Leu  Arg  Phe  Thr  Asn  Ile  Gly

Pro  Asp  Thr  Met  Arg  Val  Thr  Trp  Ala  Pro

Pro  Pro  Ser  Ile  Asp  Leu  Thr  Asn  Phe  Leu

Val  Arg  Tyr  Ser  Pro  Val  Lys  Asn  Glu  Glu

Asp  Val  Ala  Glu  Leu  Ser  Ile  Ser  Pro  Ser

Asp  Asn  Ala  Val  Val  Leu  Thr  Asn  Leu  Leu

Pro  Gly  Thr  Glu  Tyr  Val  Val  Ser  Val  Ser

Ser  Val  Tyr  Glu  Gln  His  Glu  Ser  Thr  Pro

Leu  Arg  Gly  Arg  Gln  Lys  Thr  Gly  Leu  Asp

Ser  Pro  Thr  Gly  Ile  Asp  Phe  Ser  Asp  Ile

Thr  Ala  Asn  Ser  Phe  Thr  Val  His  Trp  Ile
```

```
Ala Pro Arg Ala Thr Ile Thr Gly Tyr Arg
Ile Arg His His Pro Glu His Phe Ser Gly
Arg Pro Arg Glu Asp Arg Val Pro His Ser
Arg Asn Ser Ile Thr Leu Thr Asn Leu Thr
Pro Gly Thr Glu Tyr Val Val Ser Ile Val
Ala Leu Asn Gly Arg Glu Glu Ser Pro Leu
Leu Ile Gly Gln Gln Ser Thr Val Ser Asp
Val Pro Arg Asp Leu Glu Val Val Ala Ala
Thr Pro Thr Ser Leu Leu Ile Ser Trp Asp
Ala Pro Ala Val Thr Val Arg Tyr Tyr Arg
Ile Thr Tyr Gly Glu Thr Gly Gly Asn Ser
Pro Val Gln Glu Phe Thr Val Pro Gly Ser
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Met Ala Ile
Pro Ala Pro Thr Asp Leu Lys Phe Thr Gln
Val Thr Pro Thr Ser Leu Ser Ala Gln Trp
Thr Pro Pro Asn Val Gln Leu Thr Gly Tyr
Arg Val Arg Val Thr Pro Lys Glu Lys Thr
Gly Pro Met Lys Glu Ile Asn Leu Ala Pro
Asp Ser Ser Ser Val Val Val Ser Gly Leu
Met Val Ala Thr Lys Tyr Glu Val Ser Val
Tyr Ala Leu Lys Asp Thr Leu Thr Ser Arg
Pro Ala Gln Gly Val Val Thr Thr Leu Glu
Asn Val Ser Pro Pro Arg Arg Ala Arg Val
Thr Asp Ala Thr Glu Thr Thr Ile Thr Ile
Ser Trp Arg Thr Lys Thr Glu Thr Ile Thr
```

Gly Phe Gln Val Asp Ala Val Pro Ala Asn

Gly Gln Thr Pro Ile Gln Arg Thr Ile Lys

Pro Asp Val Arg Ser Tyr Thr Ile Thr Gly

Leu Gln Pro Gly Thr Asp Tyr Lys Ile Tyr

Leu Tyr Thr Leu Asn Asp Asn Ala Arg Ser

Ser Pro Val Val Ile Asp Ala Ser Thr Ala

Ile Asp Ala Pro Ser Asn Leu Arg Phe Leu

Ala Thr Thr Pro Asn Ser Leu Leu Val Ser

Trp Gln Pro Pro Arg Ala Arg Ile Thr Gly

Tyr Ile Ile Lys Tyr Glu Lys Pro Gly Ser

Pro Pro Arg Glu Val Val Pro Arg Pro Arg

Pro Gly Val Thr Glu Ala Thr Ile Thr Gly

Leu Glu Pro Gly Thr Glu Tyr Thr Ile Tyr

Val Ile Ala Leu Lys Asn Asn Gln Lys Ser

Glu Pro Leu Ile Gly Arg Lys Lys Thr$^{1960}$

[IV]

The present invention provides the use of the polypeptide of formula IV for the manufacture of a medicament for the therapeutic application of cancer, as claimed in claim 1.

Its method of preparation is disclosed in our Japanese Patent Application No. 131453/89 (U.S. Patent No. 5198423) , and will be explained in detail below.

The plasmid pTF7021 which codes for the polypeptide Pro$^{1239}$-Met$^{1517}$ with 279 amino acid residues is disclosed in our Japanese Laid-Open Patent Application No. 206998/89 (U.S. Patent No. 5045631). . An NcoI site is introduced immediately before the termination codon of the coding region within the plasmid pTF7021, and this results in the construction of pTF7520, which codes for Pro$^{1239}$-Ser$^{1515}$-Met.

An NcoI-HincII fragment which contains the coding region for the heparin-binding polypeptide is isolated from pHD101 and inserted into NcoI-HincII site of pTF7520, which gives a recombinant plasmid that expresses a polypeptide with 549 amino acid residues in which the cell-binding domain and the heparin-binding domain of FN are linked.

This plasmid has in its linkage site the DNA sequence that codes for the methionine residue that originated from the NcoI site and that acts as the linker. The presence of this linker does not affect the results obtained with the use of this invention, and it is possible to remove the linker readily by use of the methods of site-specific mutagenesis, when necessary.

Cells of E. coli HB101/pCH101 into which a plasmid which expresses the polypeptide Pro$^{1239}$-Ser$^{1515}$-Met-Ala$^{1690}$-Thr$^{1960}$ with 549 amino acid residues have been deposited at the Fermentation Research Institute of the Agency of Industrial Science and Technology, Japan, as FERM BP-2799.

The culture of E. coli cells which carry this plasmid under appropriate conditions causes the accumulation within the cells of the desired polypeptide.

The purification of the desired polypeptide can be done, for example, as follows. Recombinant E. coli cells are cultured in a medium such as L-broth, and the cells are collected and treated by sonication. The sonicated cell extract is centrifuged, and the supernatant is obtained. The supernatant is first dialyzed and then passed through a DEAE ion-exchange column. Steps of CM ion-exchange chromatography and/or heparin-agarose affinity chromatography, etc., are done. By these steps, it is possible to purify the desired polypeptide.

When the functional polypeptide is used in the manufacture of pharmaceutical preparation, it can be prepared in the ordinary way with a suitable known carrier for use in pharmaceutical preparations, for administration by the oral route or other routes.

As the other ingredients and as the carrier, any approved substances for pharmacologial use can be chosen, and the route of administration and the method of administration are chosen depending on their kind and on the composition. For example, for use as a liquid carrier, water, alcohols, soybean oil, olive oil, mineral oils, animal oils, vegetable oils, or synthetic oils can be used. As a solid carrier maltose, sucrose, and other sugars, amino acids, hydroxypropylcellulose and other cellulose derivatives, magnesium stearate and other salts of organic acids, etc., can be used.

When the agent is to be injectable, the liquid for the making of solution can be physiological saline, suitable buffers, solutions of sugars such as glucose, inositol, mannitol, lactose, etc., and glycols such as ethylene glycol and polyethylene glycol. In addition, sugars such as inositol, mannitol, lactose, sucrose, etc. and amino acids such as phenylalanine can be used together with a vehicle in a lyophilized preparation, and at the time of administration, the lyophilized preparation call be dissolved in an appropriate solution for use in injection, such as, for example, sterilized water, physiological saline, glucose in solution, an electrolyte solution, a solution of amino acids, or other liquids for use in intravenous administraion. The amount of the polypeptide to be incorporated in the pharmaceutical preparation depends on the preparation in question, but in general, it is preferred that the amount be 0.1-100% by weight, and more preferably, that it be 1-98% by weight. For example, when a solution for injection is being made, it is usually preferred that the amount of this polypeptide be 0.1-30% by weight, and more preferably 1-10% by weight. When administration is to be by the oral route, as well as the solid carriers or liquid carriers mentioned above, it is possible for the preparation to be in a form of a tablet, capsule, powder, granules, liquid, dry syrup, etc. In general, when the preparation is to be in the form of a capsule, granules, or powder, the amount of the active ingredient should be 5-100% by weight, and preferably 25-98% by weight.

The amount of a single dose depends on the age, weight, and signs and symptoms of the patient, and also on the purpose of treatment, etc., but in general, a therapeutic dose by a non-oral route is 1-100 mg/kg per day and by the oral route, 5-500 mg/kg per day.

Acute toxicity: When C57BL/6 mice or $CDF_1$ mice were used in a test of the toxicity of the polypeptide with 274 amino acid residues, the polypeptide with 271 amino acid residues, or the polypeptide with 549 amino acid residues, no signs of toxicity were found when 100 mg/kg of a polypeptide was given by intravenous injection.

This invention will be explained in more detail by reference to the following examples. However, this invention is not to be taken to be limited to these examples. In all of these examples, "parts" refers to "parts by weight".

Reference Example 1.

$CDF_1$ mice, in groups with 10 mice each, were injected intravenously with either $4 \times 10^4$ cells of the lymphoma cell line L5178Y-ML25 or else with a mixture of $4 \times 10^4$ cells of the lymphoma cell line of L5178-ML25 plus 250 $\mu$g of specified polypeptide of formula [II], [III] or [IV].

The prolongation of life was calculated for the groups that received one of the polypeptides as a percentage of the mean days of survival of the control group after the transplantation by intravenous injection of the lymphoma cells, as follows:

$$\text{Percent prolongation of life} = \frac{\text{Mean days of survival in group given polypeptide}}{\text{Mean days of survival of control group}} \times 100$$

Table 1 shows the number of days each mouse in each group survived, up to day 26 after transplantation of lymphoma cells, together with the survival ratio (number of mice surviving until day 26/number of mice in that group), the mean days of survival, and the percent prolongation of life.

Table 1. Anticancer effects

| Compounds | Days of survival for individual mice | Survival ratio | Mean days of survival ± SD | Prolongation of life (%) |
|---|---|---|---|---|
| Controls | 14, 15, 15, 15, 16, 16, 17, 18, 20 | 0/10 | 16.1 ± 1.8 | |
| Polypeptide with 274 amino acid residues | 15, 16, 16, 17, 17, 18, 18, 19, 20, 20 | 0/10 | 17.6 ± 1.7 | 109 |
| Polypeptide with 271 amino acid residues | 16, 18, 18, 19, 21, 21, >25, >25, >25, >25 | 4/10 | >21.3 ± 3.5 | >132 |
| Polypeptide with 549 amino acid residues | >25, >25, >25, >25, >25, >25, >25, >25, >25, >25 | 10/10 | >25.0 ± 0 | >155 |

As shown above, the administration of the polypeptides had an anticancer effect.

Reference Example 2.

(1) Some 5 x $10^5$ cells of the highly metastatic cell line B16-BL6 melanoma cells were injected in a volume of 0.05 ml subcutaneously into the foot pad of the right back foot of C57BL/6 mice, which were divided into groups of five, and 21 days after the transplantation, the primary melanoma was removed surgically.

Some mice were injected intravenously once daily with 0.1 ml of solution of one of the polypeptides of formulae [II] and [IV] dissolved at the concentration of 1 mg/ml in phosphate-buffered saline (PBS) on one of the following schedules: on days 7, 9, 11, 13, 15, 17, and 19 after the transplantation, which was before the surgery, or on days 22, 24, 26, 28, 30, 32, and 34 after the transplantation, which was after the surgery. The control mice were injected with PBS on one of these two schedules.

The mice were killed and their lungs were removed surgically on day 35 after the transplantation. The number of metastatic nodules on the lung surfaces was counted under a dissection microscope. The results are shown in Tables 2 and 3.

Table 2

| Results with administration before surgical removal of primary melanoma | |
| --- | --- |
| Compounds | Mean metastases per lung ± SD |
| Controls | 53 ± 15 |
| Polypeptide with 274 amino acid residues | 22 ± 4 |
| Polypeptide with 549 amino acid residues | 34 ± 16 |

Table 3

| Results with administration after surgical removal of primary melanoma | |
| --- | --- |
| Compounds | Mean metastases per lung ± SD |
| Controls | 106 ± 23 |
| Polypeptide with 274 amino acid residues | 43 ± 33 |
| Polypeptide with 549 amino acid residues | 32 ± 22 |

As shown above, the polypeptides inhibit the metastasis of melanoma to the lungs.

(2) $CDF_1$ mice, in groups of 5 mice each, were injected intravenously with $4 \times 10^4$ cells of the lymphoma cell line L5178Y-ML25. The cells were injected in solution together with the polypeptide with 274 amino acid residues, the polypeptide with 271 amino acid residues, or the polypeptide with 549 amino acid residues at different concentrations. The controls were injected with the lymphoma cells only.

On day 14 after the transplantation of lymphoma cells, the mice were killed, and their livers and spleens were removed, weighed, and compared. Results are in Table 4.

Table 4

| Results with administration | | | |
|---|---|---|---|
| Compounds | Dose of compound μg/mouse | Mean liver weight g ± SD | Mean spleen weight g ± SD |
| Controls | | 4.53 ± 0.50 | 0.24 ± 0.04 |
| Polypeptide with 274 amino acid residues | 100<br>250<br>500 | 3.78 ± 0.62<br>4.14 ± 0.84<br>3.84 ± 0.63 | 0.23 ± 0.03<br>0.22 ± 0.03<br>0.20 ± 0.02 |
| Polypeptide with 271 amino acid residues | 100<br>250<br>500 | 2.72 ± 0.80<br>2.24 ± 0.75<br>1.42 ± 0.35 | 0.17 ± 0.05<br>0.14 ± 0.05<br>0.11 ± 0.02 |
| Polypeptide with 549 amino acid residues | 100<br>250<br>500 | 0.90 ± 0.05<br>0.90 ± 0.07<br>0.90 ± 0.08 | 0.08 ± 0.01<br>0.08 ± 0.01<br>0.08 ± 0 |

As shown above, in the groups of mice treated with the polypeptides metastasis of lymphoma cells to the liver and the spleen was inhibited.

Comparison Example 1

To 30 parts of the polypeptide with 274 amino acid residues, PBS was added for the total weight of 2000 parts, and the solution was sterilized by filtration through a Millipore GS filter. Then 2 g of the filtrate obtained was put into a 10 ml vial and lyophilized. This gave a vial containing 30 mg of lyophilized polypeptide for use in injection.

Comparison Example 2

To 30 parts of the polypeptide with 271 amino acid residues, PBS was added for the total weight of 2000 parts, and the solution was sterilized by filtration through a Millipore GS filter. Then 2 g of the filtrate obtained was put into a 10 ml vial and lyophilized. This gave a vial containing 30 mg of lyophilized polypeptide for use in injection.

Example 1

To 30 parts of the polypeptide with 549 amino acid residues, PBS was added for the total weight of 2000 parts, and the solution was sterilized by filtration through a Millipore GS filter. Then 2 g of the filtrate obtained was put into a 10 ml vial and lyophilized. This gave a vial containing 30 mg of lyophilized polypeptide for use in injection.

Comparison Example 3

To 50 parts of the polypeptide with 274 amino acid residues, 600 parts of lactose, 330 parts of crystalline cellulose, and 20 parts of hydroxypropylcellulose were added and thoroughly mixed. The mixture was compressed in a roller-compactor, and pulverized so as to pass through meshes sized from 16-60 mesh, giving granules.

Comparison Example 4

To 50 parts of the polypeptide with 271 amino acid residues, 600 parts of lactose, 330 parts of crystalline cellulose, and 20 parts of hydroxypropylcellulose were added and thoroughly mixed. The mixture was compressed in a roller-compactor, and pulverized so as to pass through meshes sized form 16-60 mesh, giving granules.

Example 2

To 50 parts of the polypeptide with 549 amino acid residues, 600 parts of lactose, 330 parts of crylstalline cellulose, and 20 parts of hydroxypropylcellulose were added and thoroughly mixed. The mixture was compressed in a roller-compactor, and pulverized so as to pass through meshes sized from 16-60 mesh, giving granules.

The polypeptides of formulae [II], [III] and [IV] are useful as anticancer agents and are safe because they are substances related to substances present physiologically in the body. The substances can be obtained in large amounts, as the result of genetic engineering, which enhances their usefulness.

**Claims**

1.  Use of a polypeptide for the manufacture of a medicament for the therapeutic application of cancer, wherein said polypeptide has the sequence of the following Formula [IV]:

```
Pro  Thr  Asp  Leu  Arg  Phe  Thr  Asn  Ile  Gly

Pro  Asp  Thr  Met  Arg  Val  Thr  Trp  Ala  Pro

Pro  Pro  Ser  Ile  Asp  Leu  Thr  Asn  Phe  Leu

Val  Arg  Tyr  Ser  Pro  Val  Lys  Asn  Glu  Glu

Asp  Val  Ala  Glu  Leu  Ser  Ile  Ser  Pro  Ser

Asp  Asn  Ala  Val  Val  Leu  Thr  Asn  Leu  Leu

Pro  Gly  Thr  Glu  Tyr  Val  Val  Ser  Val  Ser

Ser  Val  Tyr  Glu  Gln  His  Glu  Ser  Thr  Pro

Leu  Arg  Gly  Arg  Gln  Lys  Thr  Gly  Leu  Asp

Ser  Pro  Thr  Gly  Ile  Asp  Phe  Ser  Asp  Ile

Thr  Ala  Asn  Ser  Phe  Thr  Val  His  Trp  Ile

Ala  Pro  Arg  Ala  Thr  Ile  Thr  Gly  Tyr  Arg

Ile  Arg  His  His  Pro  Glu  His  Phe  Ser  Gly

Arg  Pro  Arg  Glu  Asp  Arg  Val  Pro  His  Ser

Arg  Asn  Ser  Ile  Thr  Leu  Thr  Asn  Leu  Thr

Pro  Gly  Thr  Glu  Tyr  Val  Val  Ser  Ile  Val

Ala  Leu  Asn  Gly  Arg  Glu  Glu  Ser  Pro  Leu

Leu  Ile  Gly  Gln  Gln  Ser  Thr  Val  Ser  Asp

Val  Pro  Arg  Asp  Leu  Glu  Val  Val  Ala  Ala

Thr  Pro  Thr  Ser  Leu  Leu  Ile  Ser  Trp  Asp

Ala  Pro  Ala  Val  Thr  Val  Arg  Tyr  Tyr  Arg

Ile  Thr  Tyr  Gly  Glu  Thr  Gly  Gly  Asn  Ser

Pro  Val  Gln  Glu  Phe  Thr  Val  Pro  Gly  Ser
```

13

```
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Met Ala Ile
Pro Ala Pro Thr Asp Leu Lys Phe Thr Gln
Val Thr Pro Thr Ser Leu Ser Ala Gln Trp
Thr Pro Pro Asn Val Gln Leu Thr Gly Tyr
Arg Val Arg Val Thr Pro Lys Glu Lys Thr
Gly Pro Met Lys Glu Ile Asn Leu Ala Pro
Asp Ser Ser Ser Val Val Val Ser Gly Leu
Met Val Ala Thr Lys Tyr Glu Val Ser Val
Tyr Ala Leu Lys Asp Thr Leu Thr Ser Arg
Pro Ala Gln Gly Val Val Thr Thr Leu Glu
Asn Val Ser Pro Pro Arg Arg Ala Arg Val
Thr Asp Ala Thr Glu Thr Thr Ile Thr Ile
Ser Trp Arg Thr Lys Thr Glu Thr Ile Thr
Gly Phe Gln Val Asp Ala Val Pro Ala Asn
Gly Gln Thr Pro Ile Gln Arg Thr Ile Lys
Pro Asp Val Arg Ser Tyr Thr Ile Thr Gly
Leu Gln Pro Gly Thr Asp Tyr Lys Ile Tyr
Leu Tyr Thr Leu Asn Asp Asn Ala Arg Ser
Ser Pro Val Val Ile Asp Ala Ser Thr Ala
Ile Asp Ala Pro Ser Asn Leu Arg Phe Leu
Ala Thr Thr Pro Asn Ser Leu Leu Val Ser
Trp Gln Pro Pro Arg Ala Arg Ile Thr Gly
Tyr Ile Ile Lys Tyr Glu Lys Pro Gly Ser
Pro Pro Arg Glu Val Val Pro Arg Pro Arg
Pro Gly Val Thr Glu Ala Thr Ile Thr Gly
Leu Glu Pro Gly Thr Glu Tyr Thr Ile Tyr
Val Ile Ala Leu Lys Asn Asn Gln Lys Ser
Glu Pro Leu Ile Gly Arg Lys Lys Thr
                                    1960
```

[IV]

Patentansprüche

1. Verwendung eines Polypeptids zur Herstellung eines Arzneimittels für die therapeutische Anwendung bei Krebs, worin das genannte Polypeptid die Sequenz der folgenden Formel (IV) aufweist:

Pro Thr Asp Leu Arg Phe Thr Asn Ile Gly

Pro Asp Thr Met Arg Val Thr Trp Ala Pro

Pro Pro Ser Ile Asp Leu Thr Asn Phe Leu

Val Arg Tyr Ser Pro Val Lys Asn Glu Glu

Asp Val Ala Glu Leu Ser Ile Ser Pro Ser

Asp Asn Ala Val Val Leu Thr Asn Leu Leu

Pro Gly Thr Glu Tyr Val Val Ser Val Ser

Ser Val Tyr Glu Gln His Glu Ser Thr Pro

Leu Arg Gly Arg Gln Lys Thr Gly Leu Asp

Ser Pro Thr Gly Ile Asp Phe Ser Asp Ile

Thr Ala Asn Ser Phe Thr Val His Trp Ile

Ala Pro Arg Ala Thr Ile Thr Gly Tyr Arg

Ile Arg His His Pro Glu His Phe Ser Gly

Arg Pro Arg Glu Asp Arg Val Pro His Ser

Arg Asn Ser Ile Thr Leu Thr Asn Leu Thr

Pro Gly Thr Glu Tyr Val Val Ser Ile Val

Ala Leu Asn Gly Arg Glu Glu Ser Pro Leu

Leu Ile Gly Gln Gln Ser Thr Val Ser Asp

Val Pro Arg Asp Leu Glu Val Val Ala Ala

Thr Pro Thr Ser Leu Leu Ile Ser Trp Asp

Ala Pro Ala Val Thr Val Arg Tyr Tyr Arg

Ile Thr Tyr Gly Glu Thr Gly Gly Asn Ser

Pro Val Gln Glu Phe Thr Val Pro Gly Ser

EP 0 428 266 B1

```
Lys Ser Thr Ala Thr Ile Ser Gly Leu Lys

Pro Gly Val Asp Tyr Thr Ile Thr Val Tyr

Ala Val Thr Gly Arg Gly Asp Ser Pro Ala

Ser Ser Lys Pro Ile Ser Ile Asn Tyr Arg

Thr Glu Ile Asp Lys Pro Ser Met Ala Ile

Pro Ala Pro Thr Asp Leu Lys Phe Thr Gln

Val Thr Pro Thr Ser Leu Ser Ala Gln Trp

Thr Pro Pro Asn Val Gln Leu Thr Gly Tyr

Arg Val Arg Val Thr Pro Lys Glu Lys Thr

Gly Pro Met Lys Glu Ile Asn Leu Ala Pro

Asp Ser Ser Ser Val Val Val Ser Gly Leu

Met Val Ala Thr Lys Tyr Glu Val Ser Val

Tyr Ala Leu Lys Asp Thr Leu Thr Ser Arg

Pro Ala Gln Gly Val Val Thr Thr Leu Glu

Asn Val Ser Pro Pro Arg Arg Ala Arg Val

Thr Asp Ala Thr Glu Thr Thr Ile Thr Ile

Ser Trp Arg Thr Lys Thr Glu Thr Ile Thr

Gly Phe Gln Val Asp Ala Val Pro Ala Asn

Gly Gln Thr Pro Ile Gln Arg Thr Ile Lys

Pro Asp Val Arg Ser Tyr Thr Ile Thr Gly

Leu Gln Pro Gly Thr Asp Tyr Lys Ile Tyr

Leu Tyr Thr Leu Asn Asp Asn Ala Arg Ser

Ser Pro Val Val Ile Asp Ala Ser Thr Ala

Ile Asp Ala Pro Ser Asn Leu Arg Phe Leu

Ala Thr Thr Pro Asn Ser Leu Leu Val Ser

Trp Gln Pro Pro Arg Ala Arg Ile Thr Gly

Tyr Ile Ile Lys Tyr Glu Lys Pro Gly Ser

Pro Pro Arg Glu Val Val Pro Arg Pro Arg

Pro Gly Val Thr Glu Ala Thr Ile Thr Gly

Leu Glu Pro Gly Thr Glu Tyr Thr Ile Tyr

Val Ile Ala Leu Lys Asn Asn Gln Lys Ser

Glu Pro Leu Ile Gly Arg Lys Lys Thr
                                    1960
```

(IV)

**Revendications**

1. Utilisation d'un polypeptide pour la production d'un médicament destiné au traitement thérapeutique d'un cancer, dans laquelle ledit polypeptide possède la séquence répondant à la formule (IV) suivante :

16

Pro Thr Asp Leu Arg Phe Thr Asn Ile Gly

Pro Asp Thr Met Arg Val Thr Trp Ala Pro

Pro Pro Ser Ile Asp Leu Thr Asn Phe Leu

Val Arg Tyr Ser Pro Val Lys Asn Glu Glu

Asp Val Ala Glu Leu Ser Ile Ser Pro Ser

Asp Asn Ala Val Val Leu Thr Asn Leu Leu

Pro Gly Thr Glu Tyr Val Val Ser Val Ser

Ser Val Tyr Glu Gln His Glu Ser Thr Pro

Leu Arg Gly Arg Gln Lys Thr Gly Leu Asp

Ser Pro Thr Gly Ile Asp Phe Ser Asp Ile

Thr Ala Asn Ser Phe Thr Val His Trp Ile

Ala Pro Arg Ala Thr Ile Thr Gly Tyr Arg

Ile Arg His His Pro Glu His Phe Ser Gly

Arg Pro Arg Glu Asp Arg Val Pro His Ser

Arg Asn Ser Ile Thr Leu Thr Asn Leu Thr

Pro Gly Thr Glu Tyr Val Val Ser Ile Val

Ala Leu Asn Gly Arg Glu Glu Ser Pro Leu

Leu Ile Gly Gln Gln Ser Thr Val Ser Asp

Val Pro Arg Asp Leu Glu Val Val Ala Ala

Thr Pro Thr Ser Leu Leu Ile Ser Trp Asp

Ala Pro Ala Val Thr Val Arg Tyr Tyr Arg

Ile Thr Tyr Gly Glu Thr Gly Gly Asn Ser

Pro Val Gln Glu Phe Thr Val Pro Gly Ser

```
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Met Ala Ile
Pro Ala Pro Thr Asp Leu Lys Phe Thr Gln
Val Thr Pro Thr Ser Leu Ser Ala Gln Trp
Thr Pro Pro Asn Val Gln Leu Thr Gly Tyr
Arg Val Arg Val Thr Pro Lys Glu Lys Thr
Gly Pro Met Lys Glu Ile Asn Leu Ala Pro
Asp Ser Ser Ser Val Val Val Ser Gly Leu
Met Val Ala Thr Lys Tyr Glu Val Ser Val
Tyr Ala Leu Lys Asp Thr Leu Thr Ser Arg
Pro Ala Gln Gly Val Val Thr Thr Leu Glu
Asn Val Ser Pro Pro Arg Arg Ala Arg Val
Thr Asp Ala Thr Glu Thr Thr Ile Thr Ile
Ser Trp Arg Thr Lys Thr Glu Thr Ile Thr
Gly Phe Gln Val Asp Ala Val Pro Ala Asn
Gly Gln Thr Pro Ile Gln Arg Thr Ile Lys
Pro Asp Val Arg Ser Tyr Thr Ile Thr Gly
Leu Gln Pro Gly Thr Asp Tyr Lys Ile Tyr
Leu Tyr Thr Leu Asn Asp Asn Ala Arg Ser
Ser Pro Val Val Ile Asp Ala Ser Thr Ala
Ile Asp Ala Pro Ser Asn Leu Arg Phe Leu
Ala Thr Thr Pro Asn Ser Leu Leu Val Ser
Trp Gln Pro Pro Arg Ala Arg Ile Thr Gly
Tyr Ile Ile Lys Tyr Glu Lys Pro Gly Ser
Pro Pro Arg Glu Val Val Pro Arg Pro Arg
Pro Gly Val Thr Glu Ala Thr Ile Thr Gly
Leu Glu Pro Gly Thr Glu Tyr Thr Ile Tyr
Val Ile Ala Leu Lys Asn Asn Gln Lys Ser
Glu Pro Leu Ile Gly Arg Lys Lys Thr
                                  1960
                                  (IV)
```